(19) Europäisches Patentamt
European Patent Office
Office européen des brevets

(11) **EP 4 329 846 B1**

(12) **EUROPÄISCHE PATENTSCHRIFT**

(45) Veröffentlichungstag und Bekanntmachung des Hinweises auf die Patenterteilung:
**03.09.2025 Patentblatt 2025/36**

(21) Anmeldenummer: **22732635.2**

(22) Anmeldetag: **02.05.2022**

(51) Internationale Patentklassifikation (IPC):
*A61M 13/00* $^{(2006.01)}$ *A61M 16/16* $^{(2006.01)}$
*A61M 16/08* $^{(2006.01)}$ *A61M 16/10* $^{(2006.01)}$
*A61M 11/04* $^{(2006.01)}$

(52) Gemeinsame Patentklassifikation (CPC):
**A61M 13/003; A61M 11/042; A61M 16/0875;
A61M 16/109; A61M 16/1095; A61M 16/16;
A61M 16/161;** A61M 2205/3317; A61M 2205/3368;
A61M 2205/3389; A61M 2205/3653

(86) Internationale Anmeldenummer:
**PCT/IB2022/054025**

(87) Internationale Veröffentlichungsnummer:
**WO 2022/229935 (03.11.2022 Gazette 2022/44)**

(54) **INSUFFLATIONSVORRICHTUNG FÜR DIE LAPAROSKOPIE UND VERFAHREN ZUR BESTIMMUNG DES FEUCHTIGKEIT EINES BEFEUCHTUNGSMATERIALS**

INSUFFLATION DEVICE FOR LAPAROSCOPY AND METHOD FOR DETERMINING THE MOISTURE CONTENT OF A HUMIDIFYING MATERIAL

DISPOSITIF D'INSUFFLATION POUR LAPAROSCOPIE ET PROCEDE DE DETERMINATION DE L'HUMIDITE D'UN MATERIAU D'HUMIDIFICATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.04.2021 DE 102021002272
07.07.2021 DE 102021003506**

(43) Veröffentlichungstag der Anmeldung:
**06.03.2024 Patentblatt 2024/10**

(73) Patentinhaber: **W.O.M. World of Medicine GmbH
10587 Berlin (DE)**

(72) Erfinder: **MÜLLER, Bernd
10247 Berlin (DE)**

(74) Vertreter: **Gulde & Partner
Patent- und Rechtsanwaltskanzlei mbB
Berliner Freiheit 2
10785 Berlin (DE)**

(56) Entgegenhaltungen:
WO-A1-2008/095245   WO-A1-2015/015431
WO-A1-2017/137505   WO-A1-2019/216774
WO-A1-2020/260414   US-A1- 2017 231 278
US-A1- 2018 028 768   US-A1- 2020 329 769

## Beschreibung

Die vorliegende Erfindung betrifft einen Insufflator mit Insufflationsschlauch mit integriertem Heizelement und Befeuchtungsmaterial für die Laparoskopie, wobei eine Messung des Feuchtigkeitsgehaltes möglich ist.

## Hintergrund und Stand der Technik

Die Laparoskopie ist ein medizinischer Eingriff, bei dem die Bauchhöhle und die darin liegenden Organe visuell überprüft werden können. Hierzu werden üblicherweise kleine Hautschnitte (0,3 bis 2 Zentimeter) in die Bauchdecke gemacht und durch diese ein Trokar eingebracht, welcher wiederum eine optische Vorrichtung aufnehmen kann. Mithilfe eines speziellen Endoskops (Laparoskop) kann der Bauchraum eingesehen werden. Bei der diagnostischen Laparoskopie wird der Bauchraum lediglich visuell inspiziert, im Rahmen einer therapeutischen Laparoskopie können auch operative Eingriffe vorgenommen werden.

Üblicherweise wird zu Beginn der Laparoskopie zunächst der Bauchraum mit Gas befüllt, um ein Pneumoperitoneum zu schaffen. Hierzu sind bereits verschiedene Gase verwendet worden, wie z. B. Luft, Stickstoff oder Kohlendioxid ($CO_2$). Die Verwendung von Kohlendioxidgas hat sich besonders gut bewährt. Es wurde festgestellt, dass es, insbesondere bei längeren laparoskopischen Eingriffen sinnvoll ist, das eingeführte Gas einerseits zu erwärmen und anderseits zu befeuchten. Die Gaserwärmung dient dazu, den Patienten nicht abzukühlen, sowie ein diffuses Schmerzgefühl des Patienten zu vermeiden, welches wahrscheinlich eine Folge lokaler Abkühlung infolge des Eintritts von kaltem Gas ist. Die Befeuchtung dient dazu, einem Austrocknen der inneren Bauchoberflächen vorzubeugen, um die dabei entstehende Abkühlung zu vermeiden.

Hierzu sind im Stand der Technik bereits Anregungen gegeben. So beschreibt beispielsweise die deutsche Patentschrift DE 19510710 eine Vorrichtung, die ein Mittel zur Anpassung der Gasfeuchte vorsieht (beispielsweise einen Schwamm) und welche optional ein zusätzliches Heizelement enthalten kann.

Die DE 10 2013 000492 A1 beschreibt einen Schlauch mit integriertem Heizelement für die Laparoskopie, welcher gleichzeitig ein Befeuchtungsmittel enthält. Gemäß dieser Druckschrift wird das Befeuchtungsmittel vor einer Operation mit Wasser befeuchtet. Je nach Wasseraufnahme des dort beschriebenen Materials, dem Gasvolumenstrom und der Dauer der Operation kann intraoperativ eine Nachbefeuchtung des Befeuchtungsmittels nötig sein. Da die Verdunstungsrate des Wassers, von einer Anzahl von Parametern abhängt, kann bislang nur geschätzt werden, wann eine Nachfüllung möglich ist. Alternativ werden Ausführungen beschrieben, die einen Feuchtesensor zum Ermitteln der Gasfeuchte im Gaskanal anordnen. Damit verbinden sich allerdings mehrere Nachteile. Zum einen muss der Feuchtesensor elektrisch angebunden werden, was das Design der Filterschnittstelle komplizierter macht. Weiterhin erzeugt der Feuchtesensor einen nicht unerheblichen Strömungswiderstand im Gaskanal. Dieser führt zu einer geringeren Flussrate, die den aktuellen Flowanforderungen widerspricht.

Eine weitere Vorrichtung zur Befeuchtung von Gasen in der Medizintechnik wird in der DE 3617031A1 (Prioritäten: NZ 21263, NZ 215123 und NZ 214694) beschrieben. Im Rahmen eines aufwändig herzustellenden Schlauchsystems ist ein stets wassergefüllter Schlauch vorgesehen. Über eine mikroporöse Schlauchwand wird Wasserdampf an das Gas abgegeben. Ein Sensor überwacht die Wassertemperatur.

Eine weiterhin aus dem Stand der Technik bekannte Lösung ist, einen Feuchtesensor zu benutzen. Eine solche Lösung kann ein Sensor sein, der den Temperaturverlauf beim Aufheizen misst, wie in WO 2017/157 365 A1 beschrieben.

Eine andere Möglichkeit, die Restfeuchte ohne Feuchtesensor zu bestimmen ist in US 8,836,521 "Hydration alert" beschrieben: Die verrichtete Heizarbeit (elektrische Arbeit) wird bestimmt und genutzt, um eine Alarmierung des Anwenders zum Nachfüllen des Befeuchtungsmediums auszulösen. Dort ist ebenfalls beschrieben die Gesamtmenge an insuffliertem Aufdehnungsmedium zu nutzen, um einen Nachfüllalarm auszulösen. Nachteil der beschriebenen Lösungen ist ein hoher Aufwand für Sensoren und Vorrichtungen im Gerät, verknüpft mit einer nur indirekten Ermittlung des im Befeuchtungsgeflecht vorhandenen Befeuchtungsmediums.

EP 2388041 A1 beschreibt eine Vorrichtung, um im Rahmen einer Insufflation Arzneimittel in den Körper einzuführen. Die beschriebene Vorrichtung kann einen Feuchtigkeitssensor enthalten, der seinerseits auf einer Kapazitätsmessung basiert.

Weitere medizintechnische Vorrichtungen zur Gasbefeuchtung unter Verwendung Schläuchen, die Drähte enthalten sind in US2018/028768A1 sowie WO2008/095245A1 beschrieben. US2018/028768A1 offenbart eine Insufflationseinrichtung mit einem Insufflationsschlauch, wobei der Insufflationsschlauch mit einem spiralförmig darin eingewickelten Wulst versehen ist, wobei der Wulst ein hydrophiles oder hygroskopisches Material umfasst und so konfiguriert ist, dass er eine Flüssigkeit, z.B. Wasser, und/oder ein Medikament enthält, wobei eine Vielzahl von Heizdrähten innerhalb des Wulstes angeordnet und von diesem umgeben ist.

US 5483414 beschreibt einen Impedanzsensor zur Messung physikalischer Parameter, insbesondere der Temperatur.

Die vorliegende Erfindung soll die Nachteile der aus dem Stand der Technik bekannten Lösungen überwinden und eine Insufflationseinrichtung zur Verfügung zu stellen, die in der Lage ist, die Feuchtigkeit eines Befeuchtungsmaterials zu messen, ohne das zusätzliche Sensoren benötigt werden.

**Erfindungsgemäße Lösung**

Die Erfindung lehrt eine Ausführungsform eines Heizschlauches mit der die Permittivität des Befeuchtungsmaterials gemessen werden kann. Das Grundprinzip der erfindungsgemäßen Lösung ist ein Aufbau des Heizschlauches analog zu einem Kondensator.

Aus der Elektrotechnik sind Kondensatoren als passive Bauelemente bekannt. Kondensatoren bestehen dabei im Prinzip aus zwei elektrisch-leitfähigen Flächen (Elektroden), die von einem isolierendem Material, dem Dielektrikum, voneinander getrennt sind. Gängige Ausführungsformen derartiger Kondensatoren enthalten plattenförmige Elektroden (Kondensatorplatten). Die elektrotechnischen Eigenschaften der Kondensatoren werden neben der Fläche, dem Volumen und dem Abstand der Elektroden voneinander insbesondere durch die Permittivität des zwischen den Platten befindlichen Dielektrikums bestimmt.

Es ist ebenfalls bekannt, dass ein Kondensator bei Anlegen eines Wechselstroms einen Widerstand bildet. Dieser Wechselstromwiderstand wird auch Impedanz genannt und kann als komplexer Wechselstromwiderstand beschrieben werden. Die Impedanz kann durch Methoden gemessen werden, die im Stand der Technik beschrieben sind, insbesondere durch Widerstandsmessung eines angelegten Wechselstroms.

Die Beschreibung des Kondensators als "komplexer Widerstand" ist deswegen erforderlich, weil der nach der Formel (I)

$$(I) \qquad R = \frac{U}{I}$$

berechnete Widerstand zeitabhängig ist. Die hierzu notwendigen Berechnungsmethoden sind in den Lehrbüchern der Elektrotechnik detailliert beschrieben.

Für die vorliegende Erfindung ist relevant, dass sich die Impedanz eines Kondensators bzw. der komplexe Widerstand durch Änderung der Permittivität $\varepsilon$ des Dielektrikums ändert.

Im vorliegenden Fall besteht das Dielektrikum vor allem aus dem Befeuchtungsmaterial und dessen Wassergehalt ("Feuchtigkeit"). Aufgrund der elektrischen Leitfähigkeit von Wasser müssen die Elektroden voneinander elektrisch isoliert sein. Hierzu ist mindestens ein der Elektroden mit einer elektrischen Isolierung ummantelt.

Für den erfindungsgemäßen Anwendungszweck, nämlich die Messung der Feuchtigkeit innerhalb eines Insufflationsschlauches, der seinerseits ein Heizelement und ein befeuchtetes Befeuchtungsmaterial enthält, können im einfachsten Fall zwei Drähte als Elektroden angeordnet werden, die den oben beschriebenen Kondensator bilden. Die Drähte können innerhalb des Schlauchlumens angeordnet sein, die Drähte können insbesondere an die Schlauchinnenwand angebracht (z. B. verklebt oder verschweißt) sein. Eine Fixierung ist empfehlenswert, weil sich die Impedanz ansonsten bei der möglichen Bewegung des Heizschlauches ändern kann. In weiteren Möglichkeiten können die Drähte in die Schlauchwandung eingearbeitet (z.B. eingegossen) sein oder an der Schlauchaußenwand angebracht (z. B. angeklebt) sein. Eine Anordnung innerhalb des Schlauches ist bevorzugt. Die Drähte können dabei weitgehend gerade (parallel zur Orientierung des Schlauches) angeordnet sein. Alternativ können sie auch wendelförmig entlang der Schlauchwand angeordnet sein. Entscheidend für die erfindungsgemäße Funktion ist, dass sich das Befeuchtungsmaterial zwischen den Drähten befindet. Die beiden gegenpoligen Drähte, von denen mindestens einer isoliert sein muss, bilden auf diese Weise einen Kondensator, dessen Impedanz (bei konstanter Frequenz) von den Eigenschaften des Dielektrikums abhängt. Bei Änderung des Feuchtigkeitsgrades des Dielektrikums kommt es zu Änderungen der Impedanz, die messbar sind.

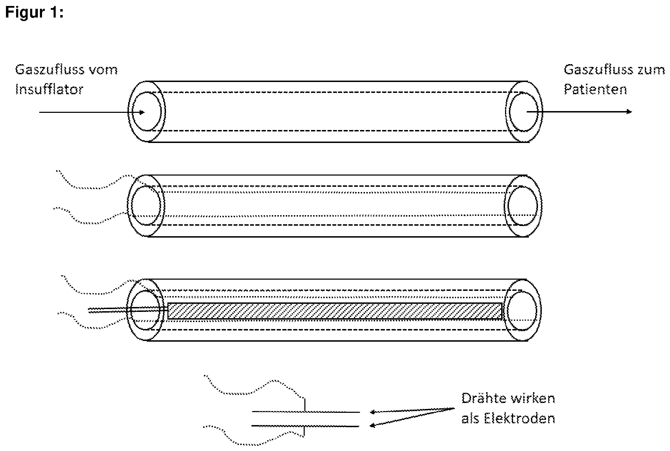

Figur 1 zeigt schematisch den Aufbau eines erfindungsgemäßen Insufflationsschlauches: Oben dargestellt ist ein leerer Schlauch. In den Schlauch werden zwei Drähte eingebracht und an der Schlauchwand fixiert (Mitte). Die Drähte dienen als Elektroden. Die Drähte (Elektroden) sind voneinander elektrisch isoliert und bilden miteinander und mit den dazwischenliegenden Medien einen Kondensator. Die Anschlüsse zum Insufflator sind dargestellt. In der unteren Abbildung sieht man das Befeuchtungsmaterial, das mit dem Heizdraht umwendelt ist. Die elektrischen Heizdrahtanschlüsse sind ebenfalls zu sehen.

Der erfindungsgemäße Insufflationsschlauch wird üblicherweise in trockenem Zustand mit dem Insufflator verbunden und der Insufflator misst zunächst die Impedanz in trockenem Zustand, das heißt mit trockenem Befeuchtungsmaterial. Anschließend wird das Befeuchtungsmaterial mit der passenden Menge des Befeuchtungsmittels (z.B. Wasser) befeuchtet. Die hierfür nötige Wassermenge hängt von der Menge des Befeuchtungsmaterials im Schlauch ab. Der Schlauch kann einen Einfüllstutzen für die Befüllung mit Wasser enthalten. Nach der Befeuchtung wird erneut die Impedanz gemessen. Im Betrieb gibt das Befeuchtungsmaterial Wasser an die Luft ab. Die Impedanz nähert sich in der Folge wieder an das Niveau des trockenen Schlauches an.

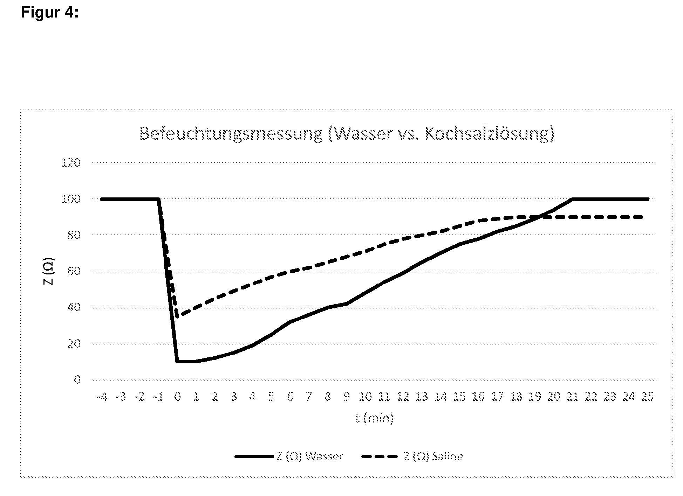

Bei Untersuchungen hat sich herausgestellt, dass einfache Metall- (z. B. Kupfer) Drähte mit einem Durchmesser von 0,1-1 mm bereits ausreichend sind, um die Änderung der dielektrischen Eigenschaften des Befeuchtungsmaterials zu messen (s.u.). Figur 4 zeigt schematisch die gemessene Änderung der Impedanz im Verlauf der Insufflationszeit.

Es hat sich herausgestellt, dass produktionsbedingte Schwankungen der Drähte (z.B. Drahtdurchmesser, -länge) oder deren Verlegung zu geringfügigen Änderungen der Impedanzen führen können. Da die Impedanz frequenzabhängig ist, können derartige Schwankungen durch Frequenzänderungen kompensiert werden. In einer speziellen Ausführungsform der Erfindung werden die Impedanzen im trockenen und/oder befeuchteten Zustand vom Insufflator gemessen und durch Änderung der Frequenz des angelegten Wechselstroms auf eine vorher bestimmte Sollimpedanz eingestellt.

In einer alternativen Ausführungsform der Erfindung werden die Impedanzen nach der Produktion im trockenen und/oder befeuchteten Zustand gemessen und die Messwerte auf einem Datenträger gespeichert. Zu den gespeicherten Daten können folgende Daten gehören:

- Impedanz (bei trockenem Befeuchtungsmaterial) bei einer oder mehreren bestimmten Messfrequenzen
- Impedanz (bei feuchtem Befeuchtungsmaterial) feucht bei einer oder mehreren bestimmten Messfrequenzen
- Messfrequenz zum Erzielen einer bestimmten Impedanz bei feuchtem Befeuchtungsmaterial
- Messfrequenz zum Erzielen einer bestimmten Impedanz bei trockenem Befeuchtungsmaterial

Die Speicherung kann beispielsweise auf einem RFID-Chip, einem Magnetband oder auf einem Barcode erfolgen, wobei der Insufflator eine kompatible Leseeinheit aufweisen muss. In diesem Fall würde der Insufflator beim oder nach dem Fixieren des Insufflationsschlauches die gespeicherten Daten auslesen und zur Einstellung des Gerätes heranziehen.

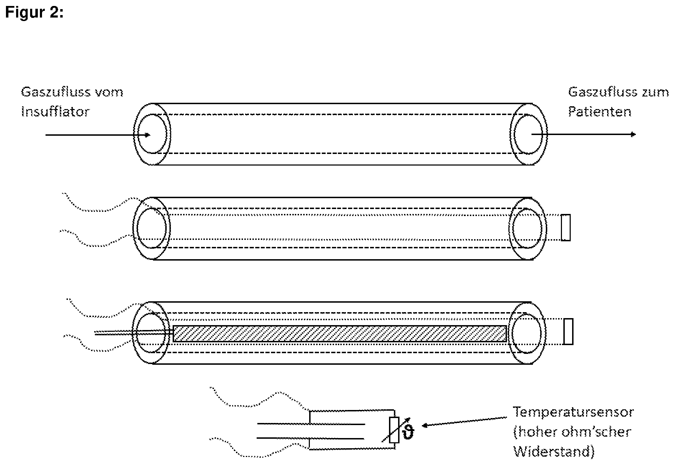

In einer anderen Ausführungsform der Erfindung werden die kondensatorbildenden Drähte durch die Anschlusskabel eines Temperatursensors gebildet. Bei dieser Ausführungsform befindet sich im Schlauch ein üblicher Temperatursensor (z. B. DS18S20). Es muss sich in jedem Fall um einen bei der zum Messen anliegenden Wechselstromfrequenz hochohmigen temperaturabhängigen Widerstand handeln, damit der Kondensator nicht kurzgeschlossen wird. Ein prinzipielles Schaltbild ist in Figur 2 dargestellt.

Bei dieser Ausführungsform der Erfindung können Temperatur- und Feuchtigkeitsgehalt nicht gleichzeitig gemessen werden, die Messung dieser Eigenschaften erfolgt nacheinander, insbesondere im stetigen Wechsel. Durch Anlegen eines Gleichstromes an die beiden Anschlusskabel kann der Widerstand des Temperatursensors gemessen werden, aus dem dann die Temperatur bestimmt wird. Durch Anlegen eines hochfrequenten Wechselstroms kann die Impedanz des gebildeten Kondensators ermittelt werden, welche in Relation zur Feuchtigkeit des Befeuchtungsmaterials steht.

Der Vorteil dieser Ausführungsform ist, dass kein zusätzlicher Sensor für die Feuchtigkeitsmessung benötigt wird. Gleichzeitig wird die Anzahl der Drähte im Schlauch begrenzt. Auch in dieser Ausführungsform können die Anschlussdrähte für den Temperatursensor außerhalb oder innerhalb des Schlauches bzw. in der Schlauchwand angeordnet sein.

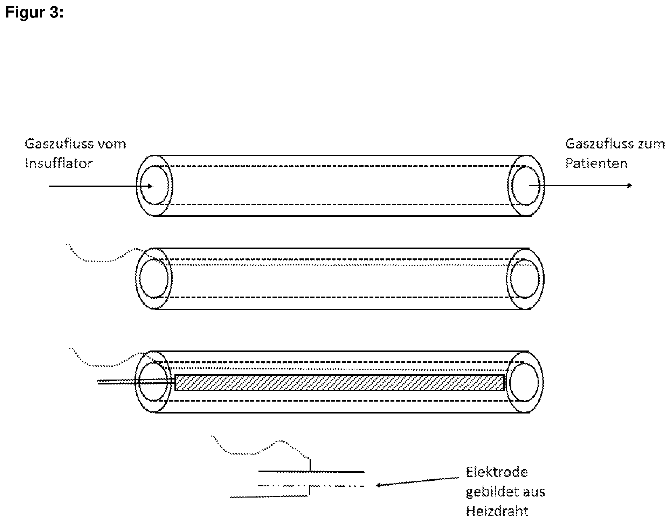

In einer weiterhin alternativen Ausführungsform der Erfindung, kann einer der kondensatorbildenden Drähte auch durch einen Heizdraht gebildet sein (Figur 3). Insufflationsschläuche mit Heizdrähten sind beispielsweise in der WO 2014/111083 A1 beschrieben worden. Auch dieser Ausführungsform ist lediglich eine zeitlich alternierende Heizung bzw. Messung möglich: Durch Anlegen eines Gleichstromes an den Heizdraht entwickelt dieser durch den Ohm'schen Widerstand Wärme, die das durchgeleitete Gas und das Befeuchtungsmaterial erwärmt. Auch hier wird der bereits oben beschriebene Vorteil verwirklicht, dass kein zusätzliches Bauelement in den Schlauch eingebracht werden kann, so dass die Messung durch Nutzung der ohnehin vorhandenen Bauteile verwirklicht werden kann. Durch Anlegen einer hochfrequenten Wechselspannung an den Heizdraht einerseits und einen parallel angeordneten Draht anderseits, kann wiederum die Impedanz des hierdurch gebildeten Kondensators bestimmt und hieraus die Feuchtigkeit des Befeuchtungsmaterials bestimmt werden.

In einer bevorzugten Ausführungsform der Erfindung erfolgt der Gaszufluss in den Insufflationsschlauch durch einen als Geflechtsschlauch ausgeformtes Befeuchtungsmaterial, das seinerseits innerhalb des Insufflationsschlauches positioniert ist. Wie in der WO 2014/111083 A1 beschrieben kann der Geflechtsschlauch auch mit dem Befeuchtungsmaterial und dem Heizdraht ummantelt sein. In jedem Fall tritt das durch den Geflechtsschlauch strömende Gas durch die Poren der Mantelfläche aus und wird dabei sowohl erwärmt, als auch befeuchtet. Bei einer Ausführungsform der Erfindung mit einem metallischen Geflechtsschlauch kann die Vergrößerung der Kondensatorfläche die Qualität der Messung positiv. beeinflussen.

Wenn es sich bei dem Heizdraht um ein Material handelt, bei dem der Widerstand mit der Temperatur steigt (z. B. ein Material mit einem positiven oder negativen Temperaturkoeffizienten (PTC oder NTC), wie in WO 2014/111084 A1 beschrieben), dann kann über die Messung des Widerstandes des Heizdrahtes auch die Temperatur gemessen werden. Die Einzelheiten einer derartigen Temperaturmessung sind in WO 2014/111084 A1 beschrieben, sodass hierauf verwiesen werden kann. Bei einer solchen Ausführungsform der Erfindung wechseln sich Heizperioden, Messperiode zur Feuchtigkeitsmessung des Befeuchtungsmaterials und Temperaturmessung periodisch ab. Die Verwendung digitaler Messsensoren in einem derartigen Aufbau ist weniger empfehlenswert, da die Versorgungsleitungen für digitale Temperatursensoren als Bussystem störanfällig durch Einstrahlung von HF-Signalen sind.

Die erfindungsgemäße Messung der Feuchtigkeit des Befeuchtungsmaterials erfolgt auf prinzipiell bekannte Weise, nämlich durch Messung der Impedanz mittels eines angelegten Wechselstroms möglichst hoher Frequenz (z. B. 100 kHz bis 100 MHz). Da die Impedanz von der genauen Bauweise der erfindungsgemäßen Vorrichtung abhängt (u. a. Abstand der kondensatorbildenden Drähte voneinander, Art des Befeuchtungsmaterials, Zusammensetzung des Befeuchtungsmittels, geometrische Anordnung der Drähte, etc.), ist für jede Bauweise eines derartigen Schlauches eine Impedanzmessung durchzuführen. Hierzu wird der weiter unten geführte Schlauchaufbau zunächst ohne Befeuchtungsmittel (Wasser) realisiert und die Impedanz des gebildeten Kondensators über übliche Methoden gemessen. Anschließend wird das Befeuchtungsmaterial durch Zugabe des Befeuchtungsmittels (Wasser) maximal befeuchtet. Unter Verwendung eines Dummys wird der Schlauch dann geheizt und von Gas durchströmt. Währenddessen wird die Impedanz kontinuierlich oder periodisch gemessen und der Verlauf der Impedanz aufgezeichnet. Auf Grund der möglichen verschiedenen Bauformen derartiger erfindungsgemäßer Insufflationsschläuche ist der Absolutwert der Impedanz vergleichsweise unwichtig. Entscheidend ist der Verlauf der Impedanz in Korrelation zu dem Befeuchtungsgrad des Befeuchtungsmaterials.

Figur 4 zeigt beispielhaft den Messzyklus und die Ergebnisse: Der beispielhaft herangezogene Schlauch wird zunächst in trockener Form vermessen und zeigt eine Impedanz von 100 Ohm. Durch Anfeuchten mit 10 ml Wasser fällt die Impedanz auf 10 Ohm. Durch das Durchleiten von Gas (hier: $CO_2$) bei gleichzeitiger Heizung auf 39°C verdampft das Wasser im Laufe der Zeit, bzw. im Laufe des Gasflusses (hier: konstanter Gasfluss von 10 l/min. Nach Durchleitung von 200l ist das Wasser praktisch vollständig verbraucht, das Befeuchtungsmaterial ist wieder trocken, so dass wieder (annähernd) die ursprüngliche Impedanz von 100 Ohm gemessen wird. Es zeigt sich, dass sich die gemessenen Absolutwerte unterscheiden, wenn statt (destilliertem) Wasser eine isotone Kochsalzlösung verwendet wird. Der Verlauf der Messkurve ist allerdings ähnlich, so dass auch in diesem Fall eine Feuchtigkeitsmessung möglich ist.

Die Aufgabe der vorliegenden Erfindung ist es vor allem, den Zustand des Befeuchtungsmaterials in Bezug auf seinen Wassergehalt, d.h. den Wassergehalt des Befeuchtungsmaterials zu ermitteln, ohne die eingangs genannten Nachteile zu implementieren. Primäre Zielstellung ist die Generierung eines Nachfüllalarms/signals, d. h. eines Signals, welches dem Benutzer mitteilt, wenn eine Nachfüllung von Wasser nötig ist.

So kann zum Beispiel ein Alarmsignal ausgelöst werden, wenn der Wassergehalt des Befeuchtungsmaterials einen voreingestellten Schwellenwert unterschreitet. Die Auslösung des Alarmsignals kann beispielsweise erfolgen, wenn ein voreingestellter Schwellenwert 50%, 40%, 30%, 20 %, 10% oder 5% der maximalen Feuchtigkeit entspricht.

Im Rahmen der vorliegenden Erfindung werden die Begriffe "Wassergehalt des Befeuchtungsmaterials" und "Feuchtigkeit des Befeuchtungsmaterials" als synonym angesehen.

**Beschreibung der Figuren**

Figur 1 zeigt schematisch den Aufbau eines erfindungsgemäßen Insufflationsschlauches: Oben dargestellt ist ein leerer Schlauch. In den Schlauch werden zwei Drähte eingebracht und an der Schlauchwand fixiert (Mitte). Die voneinander elektrisch isolierten Drähte wirken als Kondensator, dessen Impedanz von den dazwischenliegenden Medien abhängt. Die Anschlüsse zum Insufflator sind dargestellt. In der unteren Abbildung sieht man das Befeuchtungsmaterial, das mit dem Heizdraht umwendelt ist. Die elektrischen Heizdrahtanschlüsse sind ebenfalls zu sehen.

Figur 2 zeigt eine Ausführungsform der Erfindung, in der die kondensatorbildenden Drähte durch die Anschlusskabel eines Temperatursensors gebildet werden.

Figur 3 zeigt eine alternative Ausführungsform der Erfindung, bei der einer der kondensatorbildenden Drähte durch einen Heizdraht gebildet ist. In einer optionalen Variante kann die Temperatur ebenfalls durch den Heizdraht gemessen werden (analog zu WO 2014/111083 A1).

Figur 4 zeigt beispielhaft den Messzyklus und die Ergebnisse: Der beispielhaft herangezogene Schlauch wird zunächst in trockener Form vermessen und zeigt eine Impedanz von 100 Ohm. Zum Zeitpunkt *t=0* s wird mit 10 ml Wasser (destilliert) angefeuchtet, die Impedanz fällt dabei auf 10 Ohm. Durch das Durchleiten von Gas (hier: $CO_2$) bei gleichzeitiger Heizung auf 39°C verdampft das Wasser im Laufe der Zeit, bzw. im Laufe des Gasflusses (hier: konstanter Gasfluss von 10 l/min). Nach Durchleitung von 200l ist das Wasser praktisch vollständig verbraucht, das Befeuchtungsmaterial ist wieder trocken, so dass wieder (annähernd) die ursprüngliche Impedanz von 100 Ohm gemessen wird. Es zeigt sich, dass sich die gemessenen Absolutwerte unterscheiden, wenn statt (destilliertem) Wasser eine isotone Kochsalzlösung verwendet wird (gestrichelte Linie). Der Verlauf der Messkurve ist allerdings ähnlich, so dass auch in diesem Fall eine Feuchtigkeitsmessung möglich ist.

**Patentansprüche**

1. Insufflationseinrichtung zur Verwendung in der Medizintechnik enthaltend einen Insufflator zur Gasversorgung und einen Insufflationsschlauch,

   wobei der Insufflationsschlauch in seinem Inneren ein Befeuchtungsmaterial enthält, wobei das Befeuchtungsmaterial Kontakt mit einem Heizelement hat,
   wobei das Heizelement aus einem Draht besteht, der durch Anlegen eines Stroms aktivierbar ist,
   wobei der Insufflationsschlauch ferner zwei voneinander isolierte Drähte enthält, die zusammen einen Kondensator bilden, dessen Impedanz von der Feuchtigkeit des Befeuchtungsmaterials abhängt und
   wobei der Insufflator dazu ausgebildet ist:

   - an die beiden Drähte die den Kondensator bilden, eine hochfrequente Spannung anzulegen,
   - die Impedanz des Kondensators zu bestimmen, und
   - aus der Impedanz die Feuchtigkeit des Befeuchtungsmaterials zu bestimmen.

2. Vorrichtung gemäß Anspruch 1, wobei mindestens einer der elektrisch voneinander isolieren Drähte an der Außenwand des Insufflationsschlauches, innerhalb der Wand des Insufflationsschlauches, an der Innenwand des Insufflationsschlauches oder im Inneren des Insufflationsschlauches angeordnet ist.

3. Vorrichtung gemäß Anspruch 1, wobei der Insufflationsschlauch weiterhin einen Temperatursensor enthält.

4. Vorrichtung gemäß Anspruch 2, wobei der Temperatursensor am patientenseitigen Ende des Insufflationsschlauches angeordnet ist.

5. Vorrichtung gemäß Anspruch 3 und 4, wobei mindestens einer der kondensatorbildenden Drähte, durch ein Anschlusskabel des Temperatursensors gebildet wird.

6. Vorrichtung gemäß Anspruch 1, wobei mindestens einer der kondensatorbildenden Drähte, durch einen Heizdraht gebildet wird.

7. Vorrichtung gemäß Anspruch 1, wobei mindestens einer der kondensatorbildenden Drähte, durch einen metallischen Geflechtsschlauch gebildet wird.

8. Verfahren zur Messung des Wassergehaltes eines Befeuchtungsmittels, welches sich in einem Insufflationsschlauch eines Insufflators befindet, welcher von einem Gas durchströmt wird,

   wobei der Insufflationsschlauch zwei voneinander isolierte Drähte enthält,
   **dadurch gekennzeichnet, dass**
   a) an die beiden Drähte, die einen Kondensator bilden, eine hochfrequente Spannung angelegt wird
   b) die Impedanz des Kondensators bestimmt wird,
   c) aus der Impedanz die Feuchtigkeit des Befeuchtungsmaterials bestimmt wird.

9. Verfahren zur Messung des Wassergehaltes eines Befeuchtungsmittels, welches sich in einem Insufflationsschlauch eines Insufflators befindet, welcher von einem Gas durchströmt wird gemäß Anspruch 8, **dadurch gekennzeichnet, dass**

   a) an die beiden Drähte, die einen Kondensator bilden, eine hochfrequente Spannung angelegt wird
   b) die Impedanz des Kondensators mit trockenem Befeuchtungsmittel bestimmt wird,
   c) das Befeuchtungsmittel befeuchtet wird,
   d) die Impedanz des Kondensators mit feuchtem Befeuchtungsmittel bestimmt wird,
   e) der Insufflationsschlauch bestimmungsgemäß durch Durchleiten eines Gases benutzt wird,
   f) aus der Änderung der Impedanz während der Insufflation die Feuchtigkeit des Befeuchtungsmaterials bestimmt wird.

10. Verfahren gemäß Anspruch 8 oder 9, **dadurch gekennzeichnet, dass** ein Alarmsignal ausgelöst wird, wenn die Feuchtigkeit des Befeuchtungsmaterials einen voreingestellten Schwellenwert unterschreitet.

11. Verfahren gemäß Anspruch 10, **dadurch gekennzeichnet, dass** der voreingestellte Schwellenwert 50%, 40%, 30%, 20 %, 10% oder 5% der maximalen Feuchtigkeit entspricht.

12. Verfahren gemäß Anspruch 8 oder 9, **dadurch gekennzeichnet, dass** die Impedanz des Kondensators vor dem bestimmungsgemäßen Gebrauch mit trockenem und/oder feuchtem Befeuchtungsmittel durch den Insufflator durch Anlegen einer hochfrequenten Spannung gemessen wird und durch Änderung der Frequenz eine vorbestimmte Sollimpedanz mit trockenem und/oder Befeuchtungsmittel eingestellt wird, wobei die Frequenz bei der die Sollimpedanz erreicht wird für die bestimmungsgemäße Insufflation unter Gasbefeuchtung herangezogen wird.

**13.** Verfahren gemäß Anspruch 8 oder 9, **dadurch gekennzeichnet, dass** die Impedanz des Kondensators nach der Produktion mit trockenem und/oder feuchtem Befeuchtungsmittel durch Anlegen einer hochfrequenten Spannung gemessen wird und durch Änderung der Frequenz eine vorbestimmte Sollimpedanz mit trockenem und/oder Befeuchtungsmittel eingestellt wird, wobei die Frequenz bei der die Sollimpedanz erreicht wird auf einem Datenträger gespeichert wird, wobei der Datenträger durch den Insufflator ausgelesen wird und die gespeicherten Daten für die bestimmungsgemäße Insufflation unter Gasbefeuchtung herangezogen werden.

**Claims**

**1.** An insufflation device for use in medical technology containing an insufflator for gas supply and an insufflation tube,

wherein the insufflation tube contains a humidifying material in the interior thereof,
wherein the humidifying material is in contact with a heating element,
wherein the heating element consists of a wire that is activatable by application of a current,
wherein the insufflation tube further contains two wires insulated from one another that together form a capacitor, the impedance of which is dependent on the moisture of the humidifying material, and
wherein the insufflator is configured:

- to apply a high-frequency voltage to the two wires that form the capacitor,
- to determine the impedance of the capacitor, and,
- on the basis of the impedance, to determine the moisture of the humidifying material.

**2.** The device according to Claim 1, wherein at least one of the wires electrically insulated from one another is arranged on the outer wall of the insufflation tube, within the wall of the insufflation tube, on the internal wall of the insufflation tube, or in the interior of the insufflation tube.

**3.** The device according to Claim 1, wherein the insufflation tube further contains a temperature sensor.

**4.** The device according to Claim 2, wherein the temperature sensor is arranged at the patient end of the insufflation tube.

**5.** The device according to Claims 3 and 4, wherein at least one of the wires forming the capacitor is formed by a connection cable of the temperature sensor.

**6.** The device according to Claim 1, wherein at least one of the wires forming the capacitor is formed by a heating wire.

**7.** The device according to Claim 1, wherein at least one of the wires forming the capacitor is formed by a metallic braided sleeve.

**8.** A method for measuring the water content of a humidifying agent that is located in an insufflation tube of an insufflator through which a gas flows,

wherein the insufflation tube contains two wires insulated from one another,
**characterized in that**
a) a high-frequency voltage is applied to the two wires that form the capacitor,
b) the impedance of the capacitor is determined,
c) on the basis of the impedance, the moisture of the humidifying material is determined.

**9.** The method for measuring the water content of a humidifying agent that is located in an insufflation tube of an insufflator through which a gas flows according to Claim 8, **characterized in that**

a) a high-frequency voltage is applied to the two wires that form a capacitor,
b) the impedance of the capacitor is determined with the dry humidifying agent,
c) the humidifying agent is moistened,
d) the impedance of the capacitor is determined with the moist humidifying agent,
e) the insufflation tube is put to its intended use by passing a gas through it,
f) the moisture of the humidifying material is determined on the basis of the change in impedance during insufflation.

**10.** The method according to Claim 8 or 9, **characterized in that** an alarm signal is triggered if the moisture of the humidifying material falls below a preset threshold value.

**11.** The method according to Claim 10, **characterized in that** the preset threshold value corresponds to 50%, 40%, 30%, 20 %, 10%, or 5% of the maximum moisture.

**12.** The method according to Claim 8 or 9, **characterized in that** the impedance of the capacitor is measured prior to its intended use by the insufflator with a dry and/or moist humidifying agent by applying a high-frequency voltage, and a predetermined nominal impedance with dry and/or moist humidifying agent is set by modifying the frequency, wherein the frequency at which the nominal impedance is reached is used for the intended insufflation with gas humidification.

13. The method according to Claim 8 or 9, **characterized in that** the impedance of the capacitor is measured after production with a dry and/or moist humidifying agent by applying a high-frequency voltage, and a predetermined nominal impedance with dry and/or moist humidifying agent is set by modifying the frequency, wherein the frequency at which the nominal impedance is reached is saved on a data storage medium, wherein the data storage medium is read by the insufflator and the stored data is used for the intended insufflation with gas humidification.

**Revendications**

1. Dispositif d'insufflation pour utilisation dans la technique médicale, contenant un insufflateur pour l'alimentation en gaz et un tuyau d'insufflation,

dans lequel le tuyau d'insufflation contient à l'intérieur un matériau d'humidification,
dans lequel le matériau d'humidification est en contact avec un élément chauffant,
dans lequel l'élément chauffant est constitué d'un fil qui peut être activé par application d'un courant,
dans lequel le tuyau d'insufflation contient en outre deux fils isolés l'un de l'autre, qui forment ensemble un condensateur dont l'impédance dépend de l'humidité du matériau d'humidification, et
dans lequel l'insufflateur est réalisé pour :

- appliquer une tension à haute fréquence aux deux fils formant le condensateur,
- déterminer l'impédance du condensateur, et
- déterminer l'humidité du matériau d'humidification à partir de l'impédance

2. Dispositif selon la revendication 1, dans lequel au moins un des fils électriquement isolés l'un de l'autre est agencé sur la paroi extérieure du tuyau d'insufflation, à l'intérieur de la paroi du tuyau d'insufflation, sur la paroi intérieure du tuyau d'insufflation ou à l'intérieur du tuyau d'insufflation.

3. Dispositif selon la revendication 1, dans lequel le tuyau d'insufflation contient en outre un capteur de température.

4. Dispositif selon la revendication 2, dans lequel le capteur de température est agencé à l'extrémité côté patient du tuyau d'insufflation.

5. Dispositif selon les revendications 3 et 4, dans lequel au moins l'un des fils formant le condensateur est formé par un câble de raccordement du capteur de température.

6. Dispositif selon la revendication 1, dans lequel au moins l'un des fils formant le condensateur est formé par un fil chauffant.

7. Dispositif selon la revendication 1, dans lequel au moins l'un des fils formant le condensateur est formé par un tuyau métallique tressé.

8. Procédé de mesure de la teneur en eau d'un agent d'humidification qui se trouve dans un tuyau d'insufflation d'un insufflateur qui est traversé par un gaz,

le tuyau d'insufflation contenant deux fils isolés l'un de l'autre,
**caractérisé en ce que**
a) une tension à haute fréquence est appliquée aux deux fils qui forment un condensateur,
b) l'impédance du condensateur est déterminée,
c) l'humidité du matériau d'humidification est déterminée à partir de l'impédance.

9. Procédé de mesure de la teneur en eau d'un agent d'humidification qui se trouve dans un tuyau d'insufflation d'un insufflateur qui est traversé par un gaz selon la revendication 8,
**caractérisé en ce que**

a) une une tension à haute fréquence est appliquée aux deux fils qui forment un condensateur,
b) l'impédance du condensateur est déterminée avec l'agent d'humidification sec,
c) l'agent d'humidification est humidifié,
d) l'impédance du condensateur est déterminée avec l'agent d'humidification humide,
e) le tuyau d'insufflation est utilisé de manière conforme en faisant passer un gaz,
f) l'humidité du matériau d'humidification est déterminée à partir de la variation de l'impédance pendant l'insufflation.

10. Procédé selon la revendication 8 ou 9, **caractérisé en ce qu'**un signal d'alarme est déclenché lorsque l'humidité du matériau d'humidification passe en dessous d'une valeur seuil préréglée.

11. Procédé selon la revendication 10, **caractérisé en ce que** la valeur seuil préréglée correspond à 50 %, 40 %, 30 %, 20 %, 10 % ou 5 % de l'humidité maximale.

**12.** Procédé selon la revendication 8 ou 9, **caractérisé en ce que** l'impédance du condensateur est mesurée avant l'utilisation conforme par l'insufflateur avec un agent d'humidification sec et/ou humidepar application d'une tension à haute fréquence et une impédance de consigne prédéterminée est réglée avec l'agent d'humidification sec et/ou humide par modification de la fréquence, la fréquence à laquelle l'impédance de consigne est atteinte étant utilisée pour l'insufflation conforme sous humidification gazeuse.

**13.** Procédé selon la revendication 8 ou 9, **caractérisé en ce que** l'impédance du condensateur est mesurée après la production avec un agent d'humidification sec et/ou humidepar application d'une tension à haute fréquence et une impédance de consigne prédéterminée est réglée avec l'agent d'humidification sec et/ou humide par modification de la fréquence, la fréquence à laquelle l'impédance de consigne est atteinte étant mémorisée sur un support de données, le support de données étant lu par l'insufflateur et les données mémorisées étant utilisées pour l'insufflation conforme sous humidification gazeuse.

**Figur 1:**

Gaszufluss vom Insufflator

Gaszufluss zum Patienten

Drähte wirken als Elektroden

**Figur 2:**

Gaszufluss vom Insufflator

Gaszufluss zum Patienten

Temperatursensor (hoher ohm'scher Widerstand)

**Figur 3:**

Gaszufluss vom
Insufflator

Gaszufluss zum
Patienten

Elektrode
gebildet aus
Heizdraht

**Figur 4:**

Befeuchtungsmessung (Wasser vs. Kochsalzlösung)

**IN DER BESCHREIBUNG AUFGEFÜHRTE DOKUMENTE**

**In der Beschreibung aufgeführte Patentdokumente**

- DE 102013000492 A1
- DE 3617031 A1
- WO 2017157365 A1
- US 8836521 B
- EP 2388041 A1
- US 2018028768 A1
- WO 2008095245 A1
- US 5483414 A
- WO 2014111083 A1
- WO 2014111084 A1